# EUROPÄISCHE PATENTSCHRIFT

(11) **EP 1 021 954 B1**
(45) Veröffentlichungstag und Bekanntmachung des Hinweises auf die Patenterteilung: **02.10.2002**
(21) Anmeldenummer: 98124694.5
(22) Anmeldetag: 24.12.1998
(51) Int. Cl.: A01N 63/00, C12N 1/20

(54) **Rhizobakterienisolate zur Anwendung gegen phytopatogene Bodenpilze und Verfahren zur Anwendung der Rhizobakterienisolate**
Strains of rhizobacteria for use against phytopathogenic soil fungi and methods of use thereof
Isolats de rhizobactéries pour utilisation contre les champingnon phytopathogénes du sol et méthodes pour leur utilisation

(43) Veröffentlichungstag der Anmeldung: 26.07.2000
(73) Patentinhaber: Berg, Gabriele Dr., 18055 Rostock (DE); Dahl, Robert, 18182 Rövershagen (DE)
(72) Erfinder: Berg, Gabriele, Dr., 18055 Rostock (DE); Dahl, Robert, 18182 Rövershagen (DE); Kurze, Stefan, 18055 Rostock (DE)
(74) Vertreter: Gerstein, Hans Joachim, Dipl.-Ing.

(56) Entgegenhaltungen:
- WO-A-91/05475
- WO-A-92/18613
- WO-A-93/18135
- WO-A-93/19602
- WO-A-97/32973
- US-A- 5 597 565
- LEBEN ET AL.: "Effects of Pseudomonas fluorescens on Potato Plant Growth and Control of Verticillium dahliae" PHYTOPATHOLOGY, Bd. 77, Nr. 11, 1987, Seiten 1592-1595, XP002114402
- LIEVENS, KATELIJN H. ET AL: "Dominant rhizosphere bacteria as a source for antifungal agents" PESTIC. SCI. (1989), 27(2), 141-54, XP000137719
- KALBE, CLAUDIA, PETRA MARTEN,GABRIELE BERG: "Strains of the genus Serratia as beneficial rhizobacteria of oilseed rape with antifungal properties" MICROBIOL. RES., Bd. 151, Nr. 4, 1996 - 5. Mai 1996 (1996-05-05), Seiten 433-439, XP002114893
- TAHVONEN, R.: "Preliminary experiments in the use of Streptomyces spp. isolated from peat in the biological control of soil and seed-borne diseases in peat culture Phytopathogenic fungi." JOURNAL OF THE SCIENTIFIC AGRICULTURAL SOCIETY OF FINLAND = MAATALOUSTIETEELLINEN AIKAKAUSKIRJA., 1982 VOL. 54, NO. 5. P. 357-369 ILL PUBLISHER: HELSINKI: THE SOCIETY. ISSN: 0024-8835, XP002114404

## Beschreibung

Die Erfindung betrifft drei spezielle Rhizobakterienisolate zur Anwendung gegen phytopathogene Bodenpilze und ein Verfahren zur Anwendung der Rhizobakterienisolate insbesondere für Erdbeerpflanzen.

Der Erdbeeranbau hat weltweit eine große Bedeutung erlangt. 1995 wurden in Deutschland 68.800 t und 1998 etwa 78.800 t Früchte geerntet. Mit 1.155.000 t wurden 1997 in Europa 43 % der weltweiten Erträge erzielt. Zu den bedeutenden europäischen Produzenten zählen Spanien, Frankreich, Belgien, Deutschland, England, Österreich und Finnland. In den letzten Jahren war weltweit eine Zunahme der Erdbeerproduktion zu verzeichnen (1980: 1.795.000 t; 1997: 2.661.000 t; Food and Agriculture Organisation (FAO), 1998).

Die Erdbeere (*Fragaria x magna* THUILL.) ist eine mehrjährige Staude, die sich über Stolonen vermehrt. Im kommerziellen Erdbeeranbau werden Jungpflanzen (Grünoder Frigopflanzen im Spätsommer bzw. Frühjahr gesetzt. Eine Lagerung nach vorhergehender Wäsche der Wurzeln erfolgt bei einer Temperatur von ―2 °C (Frigopflanzen) über mehrere Monate.

Phytopathogene Bodenpilze können an der Erdbeere hohe Ertragsausfälle verursachen. Zahlreiche Mykosen befallen als Parasiten das vasculäre System der Wurzel und behindern den Assimilat-, Mineralstoff- und Wassertransport. Als Folge vertrocknen die oberirdischen Pflanzenorgane, während die Wurzeln verkümmern und ebenfalls absterben.

Beispiele weltweit verbreiteter Tracheomykosen sind *Phytophthora fragariae* und *Verticillium dahliae*. Sie verursachen Wurzelfäulen und Welkeerscheinungen und führen bei Erdbeerpflanzen zu Ertragsverlusten bis 80 % (DAHL, DATHE, SEEMÜLLER mdl. Mitteilungen).

Eine chemische Bekämpfungsmöglichkeit dieser Krankheiten ist in den meisten Fällen unbefriedigend; teuer *(Phytophthora fragariae)* oder nicht möglich *(Verticillium dahliae)*, da die Erreger durch die Bildung von Dauerorganen viele Jahre weit über Fruchtfolgen hinaus überdauern. In einer mit Dauerorganen kontaminierten landwirtschaftlichen Nutzfläche muß mit einem Befall der Wirtspflanzen, bedingt durch verschiedene Infektionsmodi, jederzeit gerechnet werden (AGRIOS 1998).

Symptome an durch *Phytophthora fragariae* (Symptomname: rote Wurzelfäule) erkrankten Pflanzen sind neben einem erheblichen Turgorverlust oberirdischer Organe, rot gefärbte Stengel und Mark im Übergangsbereich von der Wurzel zum Sproßansatz. Der Pilz überwintert im Boden mit Oosporen, Chlamydosporen oder Myzel an organischen Matrizen. Im Frühjahr werden Zoosporen freigesetzt und durch Bodenwasser verbreitet. Eine Kontrolle ist eingeschränkt über resistente Pflanzen, schnelltrocknende Böden, Fruchtwechsel und systemische Fungizide möglich (AGRIOS, 1998 und TASCHENBUCH DES PFLANZENARZTES, 1998).

*Verticillium dahliae* zeigt die ersten Symptome häufig erst bei der Ernte. Bei sommerlich erhöhten Temperaturen welken die Pflanzen, während sie sich bei kühler und feuchter Witterung erholen. Ältere Blätter haben an Rändern verbräunte bzw. vertrocknete Zonen, während jüngere Blätter gestaucht bleiben. In fortgeschrittenen Stadien vertrocknen ganze Pflanzen meistens nesterweise. *Verticillium dahliae* überwintert im Boden durch die Bildung von Mikrosklerotien, Myzel an Pflanzenrückständen oder in Rhizosphäre von als Zwischenwirten dienenden Wildkräutern. Es gibt nur sehr eingeschränkte Bekämpfungsmöglichkeiten über Bodenbearbeitung und Thermobehandlungen in warmen Klimaten. Fruchtfolgen eignen sich nicht für eine Bekämpfung, da gebildete Mikosklerotien im Boden über 15 Jahre überdauern (AGRIOS, 1998; FRAVEL, 1992; TASCHENBUCH DES PFLANZENARZTES, 1998). Eine Kontrolle über die Züchtung toleranter oder resistenter Sorten ist nur wenig erfolgversprechend, da die Resistenz gegen *Verticillium* wahrscheinlich multigen codiert und damit nicht absolut ist (DATHE, mdl. Mitteilung). Bisherige Fortschritte bei konventionell angewandten Züchtungsmethoden (z. B. Tango-, Sengasorten) hatten Einbußen in der Fruchtqualität zur Folge (SIMPSON, 1996).

Die Rhizosphäre zeichnet sich als wurzelnahe Bodenschicht durch erhöhte Nährstoffkonzentrationen und einen hohen Stoffumsatz aus. Als natürliches Habitat ist die Rhizosphäre von Erdbeeren durch hohe Abundanzen schädlicher, neutraler und nützlicher Organismen (Bakterien, Pilze, etc.) geprägt. Nützliche Rhizobakterien werden über ihre Funktion für die Pflanze definiert. Man kann zwischen wachstumsstimulierenden Funktionen für die Pflanze und antagonistischer Wirksamkeit gegenüber Phytopathogenen unterscheiden (BERG, 1996; LYNCH 1990). Zum Zeitpunkt der Auspflanzung ist diese mikrobielle Gemeinschaft an den gesäuberten Wurzeln des Pflanzguts gestört. Außerdem können die Wurzeln durch die Pflanzung verletzt werden, wo daß Pathogene einen Konkurrenzvorteil erlangen können und mögliche Infektionspforten geöffnet sind.

Durch den protektiven Einsatz künstlich inokulierter bakterieller Antagonisten, zu denen auch die aufgeführten Bakterienstämme gehören, wird eine in den landwirtschaftlichen Produktionsprozeß integrierte und kostengünstige Bekämpfungsmöglichkeit geboten oder erst möglich. Ein Befall der Pflanzen durch bodenbürtige Schadpilze, insbesondere *Phytophthora fragariae* und *Verticillium dahliae* ist deutlich vermindert, während das Wachstum der Pflanzen gefördert wird und der Ertrag steigt. Die Schadpilze werden durch die künstlich erhöhten Abundanzen von Antagonisten an einer Infektion der Wurzel gehindert, da sie eine "Schutzschicht" um die Wurzel aufbauen (WELLER 1998). Die Antagonisten sind über die kombinierten Mechanismen Konkurrenz, Antibiose und Lyse wirksam (CHET et al., 1990).

So ist beispielsweise in der DE-OS 195 02 065 A1 ein Isolat CON/M/91-08 der Pilzart *Coniothyrium minitans* zur Bekämpfung sklerotienbildender Pflanzenkrankheitserreger beschrieben. Es wurde erkannt, daß das Isolat eine hohe antagonistische Aktivität vor allem gegen den Krankheitserreger *S. sclerotiorum* hat, der an verschiedensten Organen von Kulturpflanzen wie Sonnenblume, Tomate, Tabak, Salat und Raps, Fäule verursacht.

In der WO 93/19602 A1 ist ein Verfahren zur Bekämpfung von Pflanzenkrankheiten, insbesondere von *Pseudocercosporella herpotrichoides*, unter Verwendung von Isolaten aus dem Bakterienstamm *Pseudomonas fluorescens* beschrieben.

Die WO-A-97/32973 beschreibt chitinolytische Enzyme aus inter alia *Steptomyces albido flavus*. Durch ihre Fähigkeit die Zellwand von Pilzen anzugreifen, können diese Chitobiosidasen als Fungizide bei Pilzbefall eingesetzt werden.

In der WO-A-93/185135 wird ein Bakterienisolat (*Streptomyces dicklowii*) beschrieben, welches nematizide und fungizide Eigenschaften besitzt.

Die US-A-5,597,565 beschreibt neue Isolate bekannter Bakterien mit antifungischer Wirkung von z. B. *Pseudomonas fluorescens* und *Serratia plymuthica*. Die Wirkung der beschriebenen Isolate richtet sich gegen Pilze wie *Botrytis cineria* und *Alternaria brassicola.* Diese Pilze rufen Lagerfäule hervor. Aufgabe der Erfindung war es, ein Isolat, ein Präparat und ein Verfahren zur Bekämpfung von pflanzenpathogenen Pilzen, insbesondere von phytopathogenen Bodenpilzen, anzugeben.

Die Aufgabe wurde durch Isolation von HRO-C48, HRO-E11 und HRO-11 aus den drei weltweit verbreiteten Gattungen *Serratia*, *Pseudomonas,* und *Streptomyces* gelöst. Zu diesen Gattungen gehören natürlich vorkommende Rhizobakterien, welche vornehmlich in der nährstoffreichen Rhizosphäre verschiedenster Kulturpflanzen siedeln.

Es hat sich herausgestellt, daß diese Isolate sowie die von den Isolaten abstammenden Mutanten und deren Erbinformationen zur Bekämpfung von pflanzenpathogenen Pilzen insbesondere bei Erdbeerpflanzen geeignet sind. Unter Erbinformatio nen werden im folgenden insbesondere Gene, Gensequenzen oder dem Genom des Isolats oder einer Mutante verstanden.

Die Isolate wurden unter folgenden Aufnahmedaten bei der Deutschen Sammlung von Mikroorganismen und Zellkulturen=DSMZ (Braunschweig, Bundesrepublik Deutschland) hinterlegt und sind für wissenschaftliche Zwecke zugänglich:

| Isolat | Bezeichnung der DSMZ |
|---|---|
| *Streptomyces albidoflavus* HRO11 | DSM 12500 |
| *Pseudomonas fluorescens* HRO-E11 | DSM 12501 |
| *Serratia plymuthica* HRO-C48 | DSM 12502 |

Der Pflanzenschutz bzw. die Pilzbekämpfung erfolgt mit einem Präparat, das eine fungizid wirksame Menge des Isolats HRO-11, HRO-E11 und/oder HRO-C48 enthält.

Vorteilhafterweise weist die fungizid wirksame Menge eine Zelldichte der Stämme von ca. 7,5E+09 cfu ml⁻¹ auf. Das Präparat ist mit abgestandenem Trinkwasser verdünnt und hat vorteilhafterweise eine Zelldichte von ca. 4E+09 cfu ml⁻¹ von allen Stämmen.

Bei einer Verwendung der Isolate bzw. der miteinander kombinierten Isolate HRO-11, HRO-E11 und/oder HRO-C48 sollte das Isolat bzw. die Isolate lebende Zellen, tote Zellen, Sporen, Pseudomyzel, Fragmenten von Pseudomyzel und/oder Kulturüberstand enthalten. Bei den Pseudomyzelen handelt es sich um kettenförmige aneinandergelagerte Blastosporen von Sproßpilzen, die einem Myzel ähnlich sind (Pschyrembel, "Klinisches Wörterbuch", 258. Auflage, Walter de Gruyter, 1998).

Zur Bekämpfung von pflanzenpathogenen Pilzen wird ein Verfahren vorgeschlagen, bei dem das Präparat auf die zu bekämpfenden Pilze, auf die Pilzorgane, auf die vor einem Pilzbefall zu schützenden Pflanzen oder Pflanzenteile, auf das Pflanzgut und/oder in bzw. auf den Boden gebracht wird.

Das Verfahren kann insbesondere zur Bekämpfung von wurzelpathogenen Tracheomykosen verwendet werden. Es hat sich als besonders geeignet zur Anwendung gegen die Pilzarten *Verticillium dahliae* und *Phytophthora fragariae* erwiesen.

Die Isolate bzw. Isolatgemische sollten formuliert sein oder als Suspension im Tauchbadverfahren angewandt werden.

Es ist vorteilhaft, wenn ein Isolatgemisch appliziert wird, das Isolate HRO-11, HRO-E11 und/oder HRO-C48 in jeweils verschiedenen Konzentrationen aufweist.

Das Isolat bzw. Isolatgemisch kann auch über Nährstoffe, Nährsubstrate oder Nährflüssigkeiten sowie über eine natürliche oder künstliche Matrix appliziert werden.

Die Erfindung wird nachfolgend anhand von Beispielen und mit den beigefügten Zeichnungen näher erläutert. Es zeigen:
- Fig. 1:: Ergebnis eines Gewächshausversuchs zur antagonistischen Wirkung von HRO-C48 und HRO-E11 gegen *Phytophthora fragariae*.
- Fig. 2:: Ergebnis eines Gewächshausversuchs zur antagonistischen Wirkung von HRO-11 zu *Phytophthora fragariae*.
- Fig. 3:: Gesamtertrag der Varianten im Jahr der Pflanzung auf einer Versuchsfläche 1;
- Fig. 4:: Gesamtertrag der Varianten im Jahr der Pflanzung auf einer Versuchsfläche 2.

Die antagonistische Wirksamkeit von vielen autochthonen Rhizobakterien "Biological Control Agents (=BCA)" und "Plant Growth Promothing Rhizobacteria (PGPR)" ist beschrieben worden. Als bakterielle Antagonisten dienten beispielsweise *Agrobacterium radiobacter* "K84" gegen *Agrobacterium tumefaciens* oder das auf *Bacillus subtilis* basierende Präparat "Quantum 4000", zur Bekämpfung bodenbürtiger Krankheiten an Getreiden und Erdnüssen. Das Präparat Mycostop kam weltweit als erstes biologisches Kontrollpräparat in den Handel. Es basiert auf *Streptomyces grisieoviri*des "K61" und wir u. a. gegen Bodenpathogene Fusariosen und *Phytophthora* ssp an Zierpflanzen und Gemüsen eingesetzt.

Das Gram positive Isolat HRO-11 wurde als Antagonist zu *Verticillium* in vitro (Dualkultur) selektiert und hat eine besonders hohe antagonistische Wirkung gegen *Phythophtora fragaria* und *Verticillium dahliae* sowie eine gute Befähigung zur Sporenbildung. Es wächst in flüssigem und festem Streptomyceten-Nährmedium DSM 65 der Deutschen Stammsammlung (DSMZ). Die Gram negativen Bakterienisolate HRO-E11 und HRO-C48 wachsen auf den für sie üblichen Agar-Nährboden bzw.

Flüssigmedien und haben ein zum Isolat HRO-11 vergleichbar hohes antagonistisches Potential gegen *Verticillium dahliae* und *Phytophthora fragariae*.

**Tabelle 1:**

| Kultureigenschaften der antagonistischen Isolate. | | | | | | |
|---|---|---|---|---|---|---|
| Bakterienart | Isolat Nr. | Nährmedium | | Inkubation [°C] | Dauer [h] | Sonstiges |
| | | flüssig (Fermentation in Batchkultur) | fest | | | |
| *Streptomyces albidoflavus* | HRO-11 | DSMZ 65 ohne Agarzusatz | DSMZ 65 mit Agarzusatz | 30 | 48 | Versporung: 5 Tage bei 4°C oder 22°C |
| *Serratia plymuthica* | HRO-C48 | DSM 1 | NA 2 | 30 | 18-24 | |
| *Pseudomonas fluorescens* | HRO-E11 | DSM 1 | NA 2 | 30 | 18-24 | |

Die erfindungsgemäß einzusetzenden Bakterienisolate können mit den jeweilig geeigneten festen und in flüssigen Nährmedien kultiviert werden (DSM 1= Nährmedium aus dem Katalog der Deutschen Stammsammlung für Mikroorganismen und Zellkulturen; NA 2= Nährträger 2, (Sifin, D)).

Die erfindungsgemäßen Mittel werden an Erdbeer Frigo- oder Grünpflanzen appliziert. Die Applikation erfolgte über 15minütiges Tauchen der Wurzeln in die Rhizobakterien-Suspension unmittelbar vor der Pflanzung. Es werden von allen Stämmen Zelldichten von ca. 4E+09 cfu ml⁻¹ appliziert. Dazu wird das handelsübliche Konzentrat von ca. 7,5E+09 cfu ml⁻¹ mit abgestandenem Trinkwasser verdünnt.

Es können folgende Pflanzenschutzbehandlungen durchgeführt werden, ohne daß eine Beeinflussung der eingesetzten biologischen Kontrollorganismen festgestellt wurde:

| | |
|---|---|
| Fungizide | Euparen gegen *Botrytis,* Systane gegen Mehltau |
| Herbizide | Venzar und Betanal |
| Acarizid | Decis |

Die Herbizidbekämpfungen wurden mehrmals im Jahr durchgeführt. Die Pflanzen wurden zweimal im Jahr mit Volldünger behandelt.
Die Erfindung soll im Folgenden im Hinblick auf einige wichtige Eigenschaften anhand von Beispielen näher charakterisiert werden:

### Beispiel 1:

### In vitro Test zur antifungischen Aktivität

Aus 6000 Isolaten der Rhizosphären von Erdbeere (*Fragaria magna* THUILL.), Raps (*Brassica napus* L.) und Kartoffel *(Solanum tuberosum* L.) wurden diese Stämme selektiert, welche in vitro eine starke antifungische Aktivität besaßen. Die Stämme wurden identifiziert, physiologisch und molekularbiologisch charakterisiert und einem Gewächshausscreening unterzogen. Mit Dualkulturen auf Agarplatten erfolgte die Testung zur antifungischen Aktivität bei den Isolaten HRO-11 (*Streptomyces albidoflavus*), HRO-C48 (*Serratia plymuthica*) und HRO-E11 (*Pseudomonas fluorescens*). Geprüft wurden die antifungischen Mechanismen Antibiose, Lysis, Siderophorensowie HCN (Cyanid) und IAA (Indol-Acetic-Acid) Produktion. Die Ergebnisse sind in der Tabelle 2 aufgelistet.

**Tabelle 2:**

| Antifungische Aktivität gegen *P. fragariae*, Resistenzinduktion und wachstumsfördernde Wirkung der zu testenden Rhizobakterien: Aktivität im Dualkulturtest gegen *V. dahliae*; +++ > 10 mm Hemmzone; Antibiose: Dünnschicht-Chromatographie (Bioautographie-Test nach BERG 1996); Lysis bezüglich Chitinase-Aktivität: Plattentest (+Hydrolyse,-keine Hydrolyse) und/oder β-1,3-Glucanase-Aktivität: nach DAUGROIS et al., 1990; Siderophore nach SCHWYN & NEILANDS, 1987 (+ 5-3 mm orange Zone); HCN= Cyanid mit "Aquaquant 14417" MERCK); IAA- Indole-Acetic-Acid nach SAWAR & KREMER, 1995. | | | | | | |
|---|---|---|---|---|---|---|
| ISOLAT | AKTIVITÄT IN DUALKULTUR | ANTIFUNGISCHE MECHANISMEN | | | RESISTENZ-INDUKTON | WACHSTUM |
| | | ANTIBIOSE | LYSI S | SIDEROPHORE | HCN | IAA |
| *Streptomyces albidoflavus HRO*-11 | +++ | + | - | + | + | + |
| *Serratia plymuthica* HRO-C48 | +++ | - | + | - | - | + |
| *Pseudomonas fluorescens* HRO-E11 | +++ | + | - | + | + | + |

Die Aktivität gegen *Verticillium dahliae* wurde im Dualkulturtest über die Größe der Hemmzone gemessen. Alle erfindungsgemäß verwendeten Isolate bilden Hemmzonen größer 10 mm (+++) aus. Über Dünnschicht-Chromatographie (Bioautographie-Test) wurde eine mögliche Antibiotikaproduktion der einzelnen Isolate untersucht. Die Isolate HRO-11 und HRO-E11 bildeten Antibiotika (+), während bei dem Isolat HRO-C48 (-) mit diesem Verfahren keine Antibiotikabildung nachgewiesen werden konnte. Eine mögliche Produktion von Siderophoren wurde ebenfalls bei den Isolaten HRO-11 und HRO-E11 festgestellt. Die Hemmhöfe bilden sich als 3-5 mm große orange gefärbte Zonen aus (+). Lysis wurde bei dem Isolat HRO-C48 nachgewiesen. Zur Analyse der lytischen Aktivität wurde die Chitinase-Aktivität oder eine β-1,3-Glucanase-Aktivität untersucht. Die Isolate HRO-11 und HRO-E11 waren lytisch nicht aktiv (-): Die Bildung von Cyanid (HCN) als Resistenz induzierendes Stoffwechselprodukt für die Pflanze wurde mit Aquaquant 14417 der Firma Merck bei den Isolaten HRO-11 und HRO-E11 detektiert. Untersuchungen zur Indole Acetic Acid (IAA)- Produktion erfolgten nach SAWAR & KREMER 1995. Alle 3 Rhizobakterien-Isolate bilden dieses pflanzliche Wachstumshormon.

Die Bakterienisolate wurden ausgewählt, da sie eine hohe Aktivität in vitro zeigten. Zudem ist allen die Verschiedenartigkeit ihrer Wirkmechanismen gemeinsam. Die Bakterien *Streptomyces albidoflavus* und *Pseudomonas fluorescens* wurden aus der Rhizosphäre der Kulturerdbeere isoliert. *Serratia plymuthica* HRO-C48 ist ein Isolat aus der Rhizosphäre von Raps. Die Herkunft der Isolate ist in der Tabelle 3 aufgelistet.

**Tabelle 3:**

| Herkunft der Isolate | | | |
|---|---|---|---|
| ISOLAT NR. | ORT | HABITAT DER ISOLATION | DATUM DER ISOLATION |
| HRO-11 | Rövershagen | *Fragaria x magna* (Kulturerdbeere) | 16.10.1995 |
| HRO-C48 | Rostock | *Brassica napus.* (Raps) | 19.07.1994 |
| HRO-E11 | Rövershagen | *Fragaria x magna* (Kulturerdbeere) | 16.10.1995 |

### Beispiel 2:

### Identifizierung der isolierten Rhizobakterien

Die Gram negativen Isolate HRO-C48 und HRO-E11 wurden mit den beiden standardisierten Testsystemen BIOLOG (Haywards, U.S.A) und nach taxonomischen Merkmalen (API) von bioMérieux (Marcy-l'Etoile, F) identifiziert (vgl. Tabelle 4). Die Identifikation von *Streptoymes albidoflavus* HRO-11 erfolgte durch Prof. Elisabeth Wellington (U.K.)

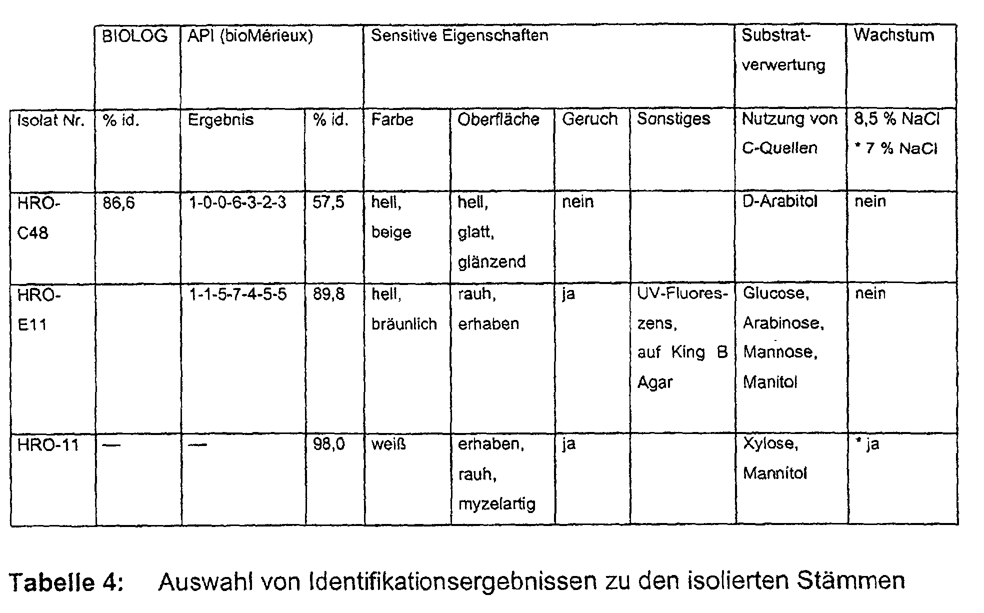

*Serratia plymuthica*, *Pseudomonas fluorescens* und *Streptomyces albidoflavus* sind der Risikogruppe 1 (ohne Risiko für die menschliche Gesundheit) zugeordnet. Sie erfüllen also die Voraussetzungen für eine mögliche Applikation im Freiland (ANONYMUS, 1990).

### Beispiel 3:

### Gewächshausversuche zur biologischen Kontrolle von Phytophthora fragariae an Erdbeeren mit den Isolaten HRO-C48, HRO-E11 und HRO-11

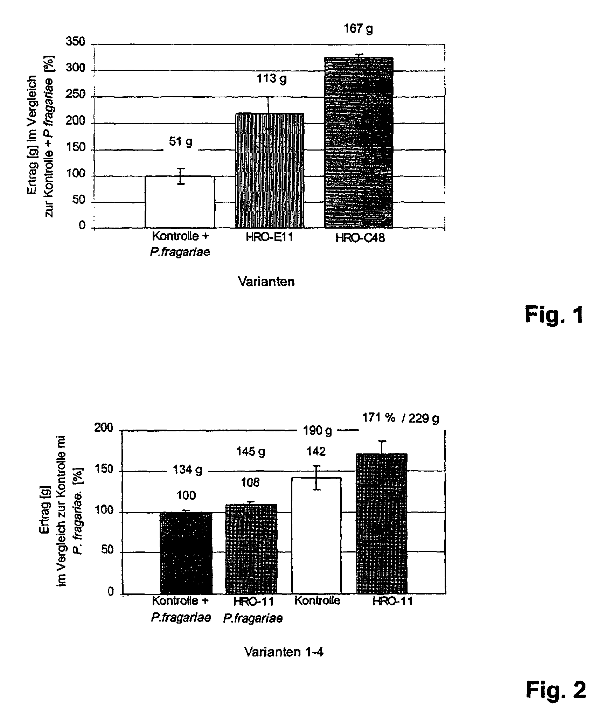

Die in vitro nachgewiesene antifungische Aktivität konnte in Gewächshausversuchen bestätigt werden, deren Ergebnisse in den Figuren 1 und 2 dargestellt sind. Nach BRÜNNER-KEINATH & SEEMÜLLER (1993) wurde dazu *Phytophthora fragariae* in einem Substrat angezogen und in Anteilen von 3 Vol.% unter die Pflanzenerde aller Varianten gemischt. Es wurden für jede Variante 3 Wiederholungen mit 10 Pflanzen angesetzt. Die endgültige Auswertung der Versuche erfolgte 12 Wochen nach Pflanzung und Inokulation über den Ernteertrag und die gesamte Biomasseproduktion.

Dargestellt sind in der Figur 1 die prozentuale Steigerung der mit den Rhizobakterien behandelten Varianten gegenüber der Kontrolle mit Phytophthora fragara (Kontrolle + *P. fragariae*). In den Balken sind die Standardabweichungen aus der vollständigen Grundgesamtheit einer Variante eingezeichnet. Der Erdbeer-Ernteertrag [g] der Variante *Serratia plymuthica* HRO-C48 lag mit 167 g 224 % über der Kontrollvariante (+ 116 g) und 54 g (+104 %) über der Variante *mit Pseudomonas fluorescens* HRO-E11. Der Ertrag von HRO-E11 war mit 113 g ca. 120 % höher als mit der unbehandelten Kontrolle.

In der Figur 1 ist das Ergebnis eines Gewächshausversuches zur antagonistischen Wirkung von HRO-C48 und HRO-E11 zu *Phytophthora fragariae* gezeigt. Dargestellt. sind die mit *P. fragariae* inokulierten und den Rhizobakterien applizierten Varianten HRO-C48, HRO-E11 und eine Kontrolle (+ *P. fragaria*) mit ihren Ernteerträgen [g]. Verglichen wird das Verhältnis der durch die Rhizobakterien verursachten Ertragssteigerung gegenüber der Kontrolle.

In der Figur 2 ist das Ergebnis eines Gewächshausversuches zur Wirkung von *Streptomyces albidoflavus* HRO-11 gezeigt. Dargestellt sind die mit *P. fragaria* inokulierte Kontrolle, dem Stamm HRO-11 applizierte Variante und eine Kontrolle ohne *P. fragariae* im Vergleich zu einer mit dem Stamm HRO-11 applizierte Variante ohne *P. fragariae* mit ihren Ernteerträgen [g]. Verglichen wird das Verhältnis der durch die Rhizobakterien verursachten Ertragssteigerung gegenüber der Kontrolle [%].

Gewächshausversuche mit dem Stamm HRO-11 konnten die Effekte zur Wachstumsstimulierung und Kontrolle von *Phytophthora fragariae* bestätigen. Die Kontrolle+P liegt mit 145 g durchschnittlich bei 8 % über der Kontrolle+ *P. fragariae*. Die Kontrolle ohne Inokulum erzielte einen Ertrag von 190 g. Dies sind 43 % (56 g) mehr, als die Kontrolle+ *P. fragariae*. Bei dem Stamm HRO-11 wurde eine wachstumsstimulierende Wirkung mit +39 g gegenüber der Kontrolle ohne Inokulum ermittelt. Die durch Vergleich der Diagramme in den Figuren 1 und 2 erkennbaren Unterschiede in den Gesamterträgen der Kontrolle+ *P. fragariae* sind auf jahreszeitlich bedingte Temperaturschwankungen der nicht klimatisierten Gewächshäuser zurückzuführen.

### Beispiel 4:

### Freilandversuche zur biologischen Kontrolle der Verticillium dahliae Welke an Erdbeeren mit den Rhizobakterien HRO-C48 und HRO-E11

In den Jahren 1997 und 1998 wurden je ein Freilandversuch auf verschiedenen Versuchsflächen durchgeführt. Dabei konnte mit den beschriebenen Isolaten die wachstumsstimulierende und antagonistische Wirkung gegenüber bodenpathogenen Tracheomykosen bestätigt werden. Die Versuchsflächen wurden bezüglich ihres phytopathogenen Potentials untersucht. Eigenen Untersuchungen nach einer internationalen Vorschrift nach TERMORSHUIZEN (1997) und einem Fremdlabor (Dresden, DAHL mdl. Mitteilungen) bestätigten eine Durchseuchung mit *Verticillium dahliae*. Bonituren und an das Pflanzenschutzamt Rostock eingesandte Proben bestätiten den in Laboruntersuchungen festgestellten hohen Infektionsdruck verschiedenster bodenpathogener Tracheomykosen auf den Versuchsflächen. Durch die einmalige Tauchbadapplikation vor der Pflanzung waren bei dem Stamm HRO-C48 nach über 12 Monaten noch antagonistische Effekte meßbar. Die Etablierung aller getesteten Stämme ließ sich ebenfalls noch nach mehreren Monaten feststellen. Bei den Stämmen HRO-C48 und HRO-E11 wurden einen Monat nach der Applikation Abundanzen von 9,00E+06 x cfu g⁻¹ (HRO-E11) isoliert. 3 Monate nach der Applikation waren die Stämme HRO-C48 mit 5,5E+03 x cfu g⁻¹ und HRO-E11 mit 1,6E+04 x cfu g⁻¹ nachweisbar. Als Kernfläche der Freilandversuche wurde etwa 1ha untersucht.

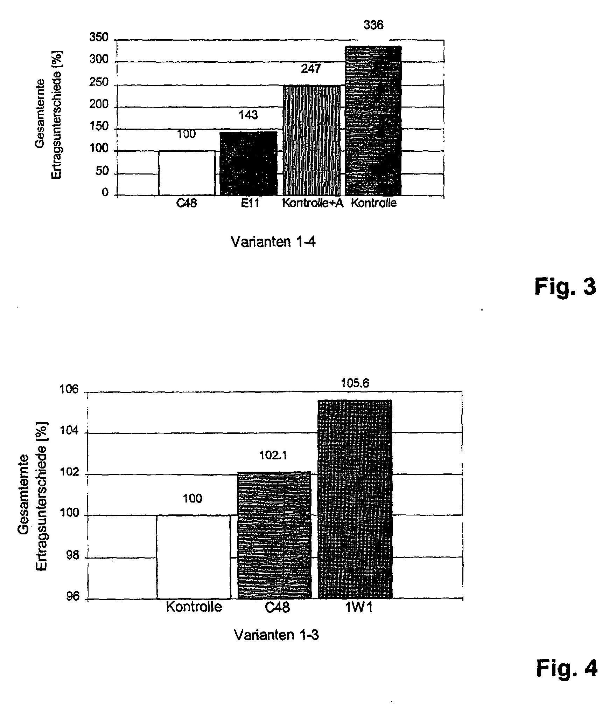

Mit den Stämmen HRO-C48 und HRO-E11 konnten im Jahr der Pflanzung (1997) auf der Versuchsfläche Nr. 1 (Standort Stuthof) deutliche Ertragssteigerungen gegenüber den Kontrollen erzielt werden, wie in dem Diagramm in der Figur 3 dargestellt ist. Als Kontrollen wurden eine unbehandelte (Kontrolle) und eine Kontrolle mit der Behandlung des systemischen Fungizids Aliette (Kontrolle+A), einem systemischen Fungizid, getestet. Die mit Aliette behandelte Kontrolle konnte sich mit einem Ertragszuwachs von 43 % gegenüber der unbehandelten Kontrolle deutlich abgrenzen. Der Stamm HRO-C48 erzielte einen um 193 % (Kontrolle+A) bzw. 136 % (Kontrolle) höheren Ertrag, die ebenfalls geringfügig erkrankten Pflanzen der Variante HRO-E11 erzielten einen Mehrertrag von 104 % (Kontrolle+A) bzw. 147 % (Kontrolle).

Die Figur 4 zeigt den Gesamtertrag der Varianten im Jahr der Pflanzung auf der Versuchsfläche Nr. 2 am Standort Goorstorf im Jahr 1998. Es werden die Varianten und deren Erträge nach den insgesamt 10 Ernten im Jahr der Pflanzung gezeigt. Der auf der zweiten Versuchsfläche durchgeführte Freilandversuch von 1998 bestätigte für das Isolat HRO-C48 die im Vorjahr auf der Versuchsfläche 1 gemessene pflanzenstärkende Wirkung. Das Gram positive Isolat von *Streptomyces albidoflavus* HRO-11 konnte eine stärkere Wirkung entfalten. HRO-C48 erzielte einen um 2,1 % höheren Ertrag als die Summe der Kontrollen (Basis 100 % = 74 kg. Das Isolat HRO-11 lag mit 105 % ca. 3,5 % über dem Ertrag der unbehandelten Kontrolle. Die im Vergleich zu 1997 (Versuchsfläche Nr. 1) geringen Abweichungen sind möglicherweise durch einen im Gesamtbestand bisher als gering eingestuften Befall von bodenbürtigen Schadpilzen (insbesondere *Verticillium dahliae*) zurückzuführen. Die im Jahr 1997 auf Versuchsfläche Nr. 1 beobachteten Größeren allgemeinen Ertragsausfälle blieben 1998 auf Versuchsfläche Nr. 1 und Nr. 2 aus. Wetterdaten (Temperatur, Niederschlagsmenge, etc.) waren während der Vegetationsperioden 1997 und 1998 sehr unterschiedlich. Durch hohe Niederschlagsmengen und geringen Hitzestreß konnten sich die jungen Frigopflanzen 1998 besser etablieren. Daher war möglicherweise die Bedeutung einer antagonistischen und wachstumsfördernden Wirkung der applizierten Varianten gegenüber Schadpilzen geringer als auf Versuchsfläche Nr. 1 (1997).

## Patentansprüche

1. Isolat HRO-11 der Bakterienart *Streptomyces albidoflavus* einschließlich jeder von diesem Isolat abstammenden Mutante und deren Erbinformationen.

2. Isolat HRO-E11 der Bakterienart *Pseudomonas fluorescens* einschließlich jeder von diesem Isolat abstammenden Mutante und deren Erbinformationen.

3. Isolat HRO-C48 der Bakterienart *Serratia plymuthica* einschließlich jeder von diesem Isolat abstammenden Mutante und deren Erbinformationen.

4. Verwendung der Isolate HRO-11, HRO-E11 und/oder HRO-C48 nach einem der Ansprüche 1 bis 3, **dadurch gekennzeichnet, daß** das Isolat aus Sporen, Pseudomyzel, lebenden Zellen, toten Zellen, Fragmenten von Pseudomyzel und/oder Kulturüberstand besteht.

5. Verwendung nach Anspruch 4, **dadurch gekennzeichnet, daß** die fungizid wirksame Menge eine Zelldichte eines Stammes oder der Stämme von im Bereich von 7,5 x 10⁹ cfu pro ml aufweist.

6. Präparat mit einem Isolat nach einem der vorhergehenden Ansprüche, **dadurch gekennzeichnet, daß** eine fungizid wirksame Menge des Isolates HRO-11, HRO-E11 und/oder HRO-C48 darin enthalten ist.

7. Präparat nach Anspruch 6, **dadurch gekennzeichnet, daß** das Präparat verdünnt ist und eine Zelldichte im Bereich von 4 x 10⁹ cfu pro ml von einem Stamm oder von allen Stämmen aufweist.

8. Präparat nach Anspruch 7, **dadurch gekennzeichnet, daß** das Präparat mit abgestandenem Trinkwasser verdünnt ist.

9. Verfahren zur Bekämpfung von pflanzenpathogenen Pilzen, **dadurch gekennzeichnet, daß** ein Präparat nach den Ansprüchen 4, 5 und 6 auf die zu bekämpfenden Pilze, auf die Pilzorgane, auf die vor einem Pilzbefall zu schützenden Pflanzen oder Pflanzenteile, auf das Pflanzgut und/oder in bzw. auf den Boden gebracht wird.

10. Verfahren nach Anspruch 9, **dadurch gekennzeichnet, daß** wurzelpathogene Tracheomykosen bekämpft werden.

11. Verfahren nach einem der Ansprüche 9 und 10, **dadurch gekennzeichnet, daß** die Pilzarten *Verticillium dahliae* und *Phytophthora fragaria* bekämpft werden.

12. Verfahren nach einem der Ansprüche 9 bis 11, **dadurch gekennzeichnet, daß** die Isolate HRO-11, HRO-E11 und/oder HRO-C48 als Suspension im Tauchbadverfahren angewandt werden.

13. Verfahren nach einem der Ansprüche 9 bis 12, **dadurch gekennzeichnet, daß** die Isolate HRO-11, HRO-E11 und HRO-C48 einzeln angewandt werden.

14. Verfahren nach einem der Ansprüche 9 bis 12, **dadurch gekennzeichnet, daß** die Isolate HRO-11, HRO-E11 und HRO-C48 kombiniert angewandt werden.

15. Verfahren nach Anspruch 14, **dadurch gekennzeichnet, daß** die Isolate HRO-11, HRO-E11 und/oder HRO-C48 in jeweils verschiedenen Konzentrationen appliziert werden.

16. Verfahren nach einem der Ansprüche 9 bis 15, **dadurch gekennzeichnet, daß** die Isolate HRO-11, HRO-E11 und/oder HRO-C48 über Nährstoffe oder Nährsubstrate oder Nährflüssigkeiten appliziert werden.

17. Verfahren nach Anspruch 10, **dadurch gekennzeichnet, daß** die Isolate HRO-11, HRO-E11 und/oder HRO-C48 über eine natürliche oder künstliche Matrix appliziert werden.

## Claims

1. Isolate HRO-11 of the bacteria type *Streptomyces albidoflavus* including all mutants derived from this isolate and their hereditary information.

2. Isolate HRO-E11 of the bacteria type *Pseudomonas fluorescens* including all mutants derived from this isolate and their hereditary information.

3. Isolate HRO-C48 of the bacteria type *Serratia plymuthica* including all mutants derived from this isolate and their hereditary information.

4. Use of the isolates HRO-11, HRO-E11 and/or HRO-C48 in accordance with one of claims 1 to 3, **characterized in that** the isolate is comprised of spores, pseudomycelium, living cells, dead cells, fragments of pseudomycelium and/or culture supernatant.

5. Use in accordance with claim 4, **characterized in that** the quantity that is sufficient to act as a fungicide possesses a cell density for a strain or strains that is in the range of 7.5 x 10⁹ cfu per ml.

6. Preparation containing an isolate in accordance with one of the preceding claims, **characterized in that** a quantity of the isolate HRO-11, HRO-E11, and/or HRO-C48 that is sufficient to act as a fungicide is contained therein.

7. Preparation in accordance with claim 6, **characterized in that** the preparation is diluted and possesses a cell density in the range of 4 x 10⁹ cfu pro ml of one strain or of all strains.

8. Preparation in accordance with claim 7, **characterized in that** the preparation is diluted with standing potable water.

9. Method for controlling plant pathogenic fungi, **characterized in that** a preparation in accordance with claims 4, 5 and 6 is applied to the fungi to be controlled, to the spores, to the plants or parts of plants to be protected against an infestation of the fungus, to the plant material, and/or in or on the soil itself.

10. Method in accordance with claim 9, **characterized in that** root-pathogenic tracheomycoses are controlled.

11. Method in accordance with one of claims 9 and 10, **characterized in that** the fungus types *Verticillium dahliae* and *Phytophthora fragaria* are to be controlled.

12. Method in accordance with one of claims 9 to 11, **characterized in that** the isolates HRO-11, HRO-E11 and/or HRO-C48 are applied as a suspension in dippling bath process.

13. Method in accordance with one of claims 9 to 12, **characterized in that** the isolates HRO-11, HRO-E11 and HRO-C48 are used individually.

14. Method in accordance with one of claims 9 to 12, **characterized in that** the isolates HRO-11, HRO-E11 and HRO-C48 are used in combination with one another.

15. Method in accordance with claim 14, **characterized in that** the isolates HRO-11, HRO-E11 and/or HRO-C48 are applied in various concentrations.

16. Method in accordance with one of claims 9 to 15, **characterized in that** the isolates HRO-11, HRO-E11 and/or HRO-C48 are applied via nutrients, nutritive substrates, or nutritive fluids.

17. Method in accordance with claim 10, **characterized in that** the isolates HRO-11, HRO-E11 and/or HRO-C48 are applied via a natural or artifical matrix.

## Revendications

1. Isolat HRO-11 de l'espèce bactérienne *Streptomyces albidoflavus*, y compris tous les mutants dérivant de cet isolat et leur information génétique.

2. Isolat HRO-E11 de l'espèce bactérienne *Pseudomonas fluorescens*, y compris tous les mutants dérivant de cet isolat et leur information génétique.

3. Isolat HRO-C48 de l'espèce bactérienne *Serratia plymuthica*, y compris tous les mutants dérivant de cet isolat et leur information génétique.

4. Utilisation des isolats HRO-11, HRO-E11 et/ou HRO-C48 selon l'une des revendications 1 à 3, **caractérisée en ce que** l'isolat se compose de spores, de pseudomycélium, de cellules vivantes, de cellules mortes, de fragments de pseudomycélium et/ou de surnageant de culture.

5. Utilisation selon la revendication 4, **caractérisée en ce que** la quantité à action fongicide présente une densité cellulaire d'une souche ou des souches de l'ordre de 7,5 x 10⁹ cfu par ml.

6. Préparation faite avec un isolat selon l'une des revendications précédentes, **caractérisée en ce qu'**elle contient une quantité à action fongicide de l'isolat HRO-11, HRO-E11 et/ou HRO-C48.

7. Préparation selon la revendication 6, **caractérisée en ce que** la préparation est diluée et présente une densité cellulaire de l'ordre de 4 x 10⁹ cfu par ml d'une souche ou de toutes les souches.

8. Préparation selon la revendication 7, **caractérisée en ce que** la préparation est diluée avec de l'eau potable éventée.

9. Procédé pour combattre les champignons phytopathogènes, **caractérisé en ce qu'**on applique une préparation selon les revendications 4, 5 et 6 sur les champignons à combattre, sur les organes des champignons, sur les plantes ou parties de plantes à protéger contre une attaque des champignons, sur la matière végétale respectivement et/ou sur le sol.

10. Procédé selon la revendication 9, **caractérisé en ce qu'**il est destiné à combattre les trachéomycoses pathogènes pour les racines.

11. Procédé selon l'une des revendications 9 et 10, **caractérisé en ce qu'**il est destiné à combattre les espèces de champignons *Verticillium dahliae* et *Phytophthora fragaria*.

12. Procédé selon l'une des revendications 9 à 11, **caractérisé en ce qu'**on applique les isolats HRO-11, HRO-E11 et/ou HRO-C48 sous forme de suspension par un procédé d'immersion.

13. Procédé selon l'une des revendications 9 à 12, **caractérisé en ce qu'**on applique les isolats HRO-11, HRO-E11 et HRO-48 isolément.

14. Procédé selon l'une des revendications 9 à 12, **caractérisé en ce qu'**on applique les isolats HRO-11, HRO-Z11 et HRO-C48 de façon combinée.

15. Procédé selon la revendication 14, **caractérisé en ce qu'**on applique les isolats HRO-11, HRO-E11 et/ou HRO-C48 à des concentrations à chaque fois différentes.

16. Procédé selon l'une des revendications 9 à 15, **caractérisé en ce qu'**on applique les isolats ERO-11, HRO-E11 et/ou HRO-C48 sur des aliments ou des substrats ou liquides alimentaires.

17. Procédé selon la revendication 10, **caractérisé en ce qu'**on applique les isolats HRO-11, HRO-E11 et/ou HROC48 sur une matrice naturelle ou synthétique.
